# DEMANDE DE BREVET EUROPEEN

(11) **EP 4 248 764 A1**
(43) Date de publication de la demande: **27.09.2023**
(21) Numéro de dépôt: 23163221.7
(22) Date de dépôt: 21.03.2023
(51) Int. Cl.: A23L 33/105, A23L 33/15, A23L 2/52, A61K 36/38, A61K 36/82, A61P 25/22, A61P 25/26

(54) **COMPOSITION COMPRENANT UN EXTRAIT DE BACOPA MONNIERI ET DU MATCHA**

(30) Priorité: 21.03.2022 FR 2202484
(71) Demandeur: Nootropia, 94160 Saint Mandé (FR)
(72) Inventeur: SELLAME, Anabelle, 94160 Saint Mandé (FR)
(74) Mandataire: Icosa

(57) **Abrégé**

La présente invention concerne une composition nutraceutique comprenant du *Bacopa monnieri* et du matcha. La présente invention concerne également ladite composition nutraceutique pour son utilisation en tant que complément alimentaire, notamment pour augmenter l'énergie et les fonctions cognitives d'un sujet, en particulier sa concentration et sa mémoire, tout en diminuant le stress et l'anxiété. Enfin, la présente invention concerne aussi un procédé de préparation d'une solution buvable de ladite composition nutraceutique.

## Description

### DOMAINE DE L'INVENTION

La présente invention concerne une composition nutraceutique comprenant du *Bacopa monnieri* et du matcha. La présente invention concerne également ladite composition nutraceutique pour son utilisation en tant que complément alimentaire, notamment pour augmenter l'énergie et les fonctions cognitives d'un sujet, en particulier sa concentration et sa mémoire, tout en diminuant le stress et l'anxiété. Enfin, la présente invention concerne aussi un procédé de préparation d'une solution buvable de ladite composition nutraceutique.

### ÉTAT DE LA TECHNIQUE

Pour pouvoir accomplir de manière plus efficace les tâches associées aux contraintes de la vie quotidienne et du milieu professionnel, différentes méthodes permettant une augmentation d'énergie et des fonctions cognitives peuvent être employées. Par exemple, la consommation d'un stimulant comme la caféine permet de stimuler le système nerveux central ce qui entraine un maintien en alerte et une diminution de la somnolence. Cependant, la caféine agit pendant une à deux heures seulement et lorsque son effet prend fin, le sujet peut ressentir une fatigue et une torpeur affectant sa productivité (« coup de barre »). Par ailleurs, la caféine entraine une augmentation du niveau de cortisol (hormone du stress) (Lovallo et al., Pharmacology, Biochemistry and Behaviour, 2006, 83(3), 441-447). Actuellement, la grande majorité des boissons énergisantes disponibles sur le marché ont pour ingrédient principal la caféine. Leur composition comprend aussi d'autres ingrédients qui peuvent entraîner des effets indésirables chez le consommateur, notamment au niveau du système cardiovasculaire (Ali et al., Postgraduate Medical Journal, 2015, 127(3), 308-322).

Il existe donc un besoin de développer des compositions alternatives comprenant des ingrédients n'ayant pas d'effet indésirable pour le consommateur et permettant d'augmenter l'énergie et les fonctions cognitives du sujet (notamment sa concentration et sa mémoire) tout en procurant un effet calmant, en particulier une diminution du stress et de l'anxiété.

Le matcha est une poudre de thé vert moulu qui possède des propriétés exclusives résultant du traitement particulier appliqué au théier : il est protégé de la lumière pendant plusieurs jours voire plusieurs semaines avant la récolte des feuilles. Le matcha comprend des taux élevés de caféine, entre 18,9 et 44,4 mg/g (Kochman et al., Molecules, 2020, 26(1), 85). La plante *Bacopa monnieri* de la famille des *scrophulariaceae* est un nootrope qui est utilisé traditionnellement en médecine ayurvédique.

Les inventeurs ont découvert des effets surprenants lorsque le matcha est associé à un extrait de *Bacopa monnieri* : une augmentation de l'énergie et des fonctions cognitives du sujet (notamment sa concentration et sa mémoire) tout en procurant un effet calmant, en particulier une diminution du stress et de l'anxiété.

### RÉSUMÉ

La présente invention concerne une composition nutraceutique comprenant :
- du *Bacopa monnieri,* et
- du matcha.

De préférence, l'invention concerne une composition nutraceutique comprenant
- un extrait de *Bacopa monnieri,* et
- du matcha.

Encore mieux, l'invention concerne une composition nutraceutique comprenant
- entre 0,16 % et 0,92 % en poids d'un extrait de *Bacopa monnieri* par rapport au poids total de ladite composition, et
- entre 0,12 % et 0,91 % en poids de matcha par rapport au poids total de ladite composition.

Dans un mode de réalisation, la composition nutraceutique selon l'invention comprend en outre un extrait de *Rhodiola rosea* et/ou du Yerba Maté.

Dans un mode de réalisation, la composition nutraceutique selon l'invention comprend en outre au moins un principe actif additionnel choisi dans le groupe constitué de : caféine, L-théanine, lutéine, zéaxanthine, *Panax ginseng*, *Curcuma longa*, Cordyceps, Ashwaganda, créatine, *Panax quinquefolius,* zinc, *Ocimum sanctum,* safran, *Ginkgo biloba,* extrait *d'Andrographis paniculata,* cannabidiol (CBD), riboflavine (vitamine B2), biotine (vitamine B8), acide folique (vitamine B9), vitamine A et vitamine E.

Dans un mode de réalisation, la composition nutraceutique selon l'invention comprend entre 0,16 % et 0,83 % en poids, préférentiellement entre 0,16 % et 0,7 % en poids, et plus préférentiellement entre 0,2 % et 0,7 % en poids d'extrait de *Bacopa monnieri* par rapport au poids total de ladite composition.

Dans un mode de réalisation, la composition nutraceutique selon l'invention comprend entre 0,12 % et 0,83 % en poids, préférentiellement entre 0,12 % et 0,7 % en poids, et plus préférentiellement entre 0,5 % et 0,7 % en poids de matcha par rapport au poids total de ladite composition.

Dans un mode de réalisation, la composition nutraceutique selon l'invention comprend entre 0,092 % et 0,58 % en poids, préférentiellement entre 0,25 % et 0,42 % en poids et plus préférentiellement entre 0,3 % et 0,36 % en poids d'extrait de *Rhodiola rosea* par rapport au poids total de ladite composition.

Dans un mode de réalisation, la composition nutraceutique selon l'invention comprend entre 0,5 % et 3,33 % en poids, préférentiellement entre 1 % et 2 % en poids et plus préférentiellement entre 1,3 % et 1,7 % en poids de Yerba Maté, de préférence sous la forme de feuilles sèches de Yerba Maté, par rapport au poids total de ladite composition.

Dans un mode de réalisation, la composition nutraceutique selon l'invention comprend en outre au moins un excipient choisi dans le groupe constitué d'extrait naturel aromatique de pêche, d'extrait naturel aromatique d'abricot, d'huile essentielle de menthe des champs, de miel, de stévia, de sirop de dattes, de chlorophylle cuivrique, de pectine, d'agar agar, d'hydroxypropylméthylcellulose, de cellulose, d'acide stéarique, de cellulose microcristalline, de maltitol, de sorbitol, de mannitol, de polyéthylène glycol, de sucralose, de polysorbate 80, de diméthylpolysiloxane, de sorbate de potassium, de benzoate de sodium et d'acide citrique.

La présente invention porte également sur la composition nutraceutique selon l'invention pour son utilisation en tant que complément alimentaire, de préférence pour augmenter l'énergie et les fonctions cognitives d'un sujet tout en diminuant le stress et l'anxiété.

La présente invention porte en outre sur un procédé de préparation d'une composition nutraceutique selon l'invention, ladite composition nutraceutique étant une solution buvable, caractérisé en ce qu'il comprend les étapes suivantes :
(a) préparer un milieu véhicule liquide,
(b) mélanger ensemble entre 0,16 % et 0,92 % en poids d'un extrait de *Bacopa monnieri* par rapport au poids total de ladite composition et entre 0,12 % et 0,91 % en poids de matcha par rapport au poids total de ladite composition,
(c) ajouter de manière progressive le milieu véhicule liquide obtenu à l'étape a) sur le mélange obtenu à l'étape b),
(d) éventuellement, ajouter un extrait de *Rhodiola rosea* et/ou du Yerba Maté,
(e) éventuellement ajouter au moins un principe actif additionnel tel que décrit ci-dessus et/ou au moins un excipient tel que décrit ci-dessus, et
(f) mélanger l'ensemble obtenu à l'étape c) ou aux éventuelles étapes d) ou e) pour obtenir la composition nutraceutique.

### DÉFINITIONS

Dans la présente invention, les termes ci-dessous sont définis de la manière suivante :
"**Acide citrique**" désigne le composé chimique de formule : 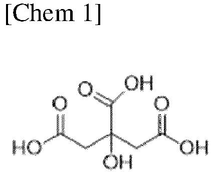
"**Acide folique**" ou "**vitamine B9**" désigne le composé chimique de formule : 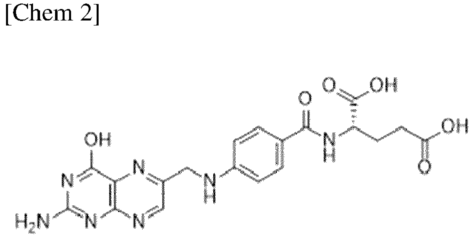
"**Administration**" signifie prendre (ou faire prendre à un patient) un complément alimentaire, et/ou un médicament, et/ou tout autre type de remède.
"**Administration par voie orale**" désigne une administration dans la cavité buccale, suivie par l'ingestion d'un composé, qui rejoint la circulation systémique à la suite de son absorption intestinale.
"***Andrographis paniculata***" désigne une plante de la famille des *Acanthaceae.*
"**Antioxydant**" désigne une molécule qui ralentit ou empêche le processus d'oxydation, c'est-à-dire l'oxydation d'autres substances chimiques en particulier due à la présence de radicaux libres en les neutralisant.
"**Ashwaganda**" ou "***Withania somnifera***" désigne une plante de la famille des *Solanaceae.*
"**Benzoate de sodium**" désigne le composé chimique de formule : 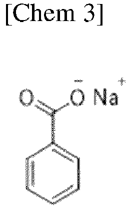
"**Biotine**" ou "**vitamine B8**" désigne le composé chimique de formule : 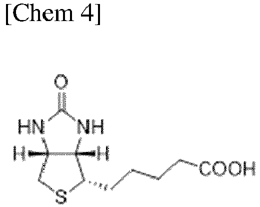
"**Caféine**" désigne le composé chimique de formule : 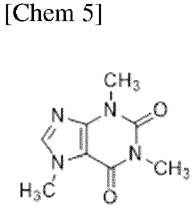
"**Cannabidiol**" (CBD) désigne un cannabinoïde de formule : 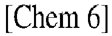 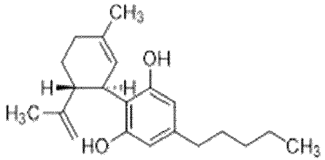
"**Complément alimentaire**" désigne une denrée alimentaire dont le but est de compléter un régime alimentaire normal et qui constitue une source concentrée de nutriments ou d'autres substances ayant un effet nutritionnel ou physiologique.
"**Comprendre**" et ses déclinaisons ont dans la présente demande un caractère non limitatif. En d'autres termes, lorsqu'une composition comprend des composés, cela n'exclut pas la présence d'autres composés que ceux mentionnés dans la composition. « Comprendre » et ses déclinaisons englobent également des expressions plus étroites telles que « être constitué essentiellement de ».
"**Constituer de**" doit être interprété dans un sens étroit, non inclusif, limité aux caractéristiques qui suivent ce terme.
"**Compris entre X et Y**" désigne la gamme de valeurs comprises entre X et Y, les bornes X et Y étant incluses dans ladite gamme, sauf précision contraire.
"**Cordyceps**" désigne un champignon de la famille des *Cordycipitaceae.*
"**Créatine**" désigne le composé chimique de formule : 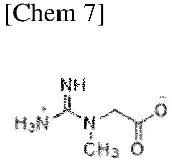
"***Curcuma longa***" désigne une plante de la famille des *Zingiberaceae.*
"**Environ**" ou "**approximativement**", placé devant un chiffre ou un nombre, signifie plus ou moins 10 % de la valeur nominale de ce chiffre ou ce nombre, de préférence plus ou moins 5 % de ladite valeur nominale, en particulier plus ou moins 1 % de ladite valeur nominale.
"**Excipient**" désigne une substance sans activité thérapeutique qui par son addition dans une composition contenant un produit d'intérêt permet d'obtenir une consistance donnée ou d'autres caractéristiques physiques particulières, comme par exemple des propriétés organoleptiques, pharmacodynamiques ou pharmacocinétiques.
"**Extrait de plante**" (par exemple, extrait de *Bacopa monnieri* et extrait de *Rhodiola rosea*) désigne la préparation obtenue après trempage d'une plante ou d'une partie de plante dans un liquide (par exemple comprenant un solvant), filtration et retrait dudit liquide.
"**Fonctions cognitives**" désigne les capacités mentales telles que la perception, l'attention, la mémoire, la concentration ou encore le langage.
"***Ginkgo biloba***" désigne une plante de la famille des *Ginkgoaceae.*
"**Guarana**" désigne une plante ("*Paullinia cupana*") de la famille des *Sapindaceae.*
"**Huile essentielle**" (par exemple, huile essentielle de menthe des champs) désigne un concentré de substances volatiles d'une plante obtenu par différentes méthodes (distillation à la vapeur d'eau, extraction mécanique, ...).
"**Ingrédient**" désigne un produit qui entre dans la composition.
"**L-théanine**" désigne l'acide aminé de formule : 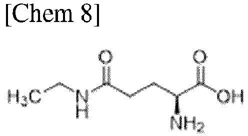
"**Lutéine**" désigne le composé chimique de formule : 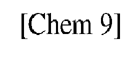 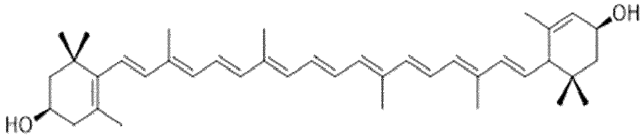
"**Milieu véhicule** " désigne un milieu dans lequel les principes actifs principaux et additionnels, ainsi que les éventuels excipients définis ci-après sont portés, de préférence dilués. Le milieu véhicule dans la présente invention peut être sous forme sèche (milieu véhicule sec) ou sous forme liquide ou de gel. De préférence, le milieu véhicule est un milieu véhicule sous forme liquide ou de gel et est appelé milieu véhicule liquide. Lorsque le milieu véhicule est sous forme liquide, le milieu peut par exemple être une solution, de préférence buvable et/ou aqueuse. Le terme "**gel**" désigne un réseau tridimensionnel de molécules liées de manière covalente ou non covalente (formant une matrice) dans un liquide (qui agit comme un agent gonflant). Le gel présente les propriétés d'un solide, allant de la ductilité à la fragilité. Un gel a une viscosité égale ou supérieure à 5.10-3 Pa.s à une température de 25°C et une pression de 1 atm, contrairement à un liquide dont la viscosité est inférieure à 5.10-3 Pa.s à une température de 25°C et une pression de 1 atm.
"**Nootrope** " ou encore "**nootropique**" est une substance qui permet d'augmenter les fonctions cognitives d'un individu après administration et qui n'est pas nocif pour la santé à dose standard.
"***Ocimum sanctum***" ou "***Ocimum tenuiflorum***" désigne une plante de la famille des *Lamiaceae.*
"***Panax ginseng***" désigne une plante de la famille des *Araliaceae.*
"*P**anax quinquefolius***" désigne une plante de la famille des *Araliaceae.*
"**Plante**" désigne la plante en tant que telle, mais aussi des parties de la plante telles que les fruits, les feuilles, les graines, les tiges, les racines, les fleurs, etc... Ce terme désigne aussi la plante ou les parties de la plante sous forme hachée, sous forme moulue, sous forme de poudre, etc...
"**Principe actif**" désigne un produit qui entre dans la composition et qui possède des propriétés particulières.
"***Rhodiola rosea***" désigne une plante de la famille des *Crassulaceae.*
"**Riboflavine**" ou "**vitamine B2**" désigne le composé chimique de formule : 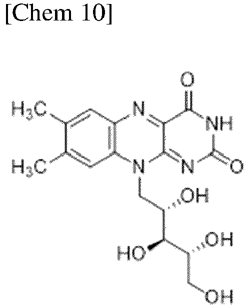
"**Safran**" désigne une épice issue de la fleur de la plante *Crocus sativus* de la famille des *Iridaceae.*
"**Sorbate de potassium**" désigne le composé chimique de formule : 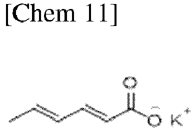
"**Stimulant**" désigne une substance responsable d'une augmentation du niveau d'éveil et/ou des activités motrices, et/ou une diminution de la somnolence d'un sujet.
"**Sujet**" fait référence à un animal, y compris un être humain. Au sens de la présente invention, un sujet peut être une personne dont l'objectif est d'augmenter son énergie et ses fonctions cognitives tout en ressentant un effet calmant, en particulier une diminution du stress et de l'anxiété.
"**Thé vert**" désigne les feuilles séchées du théier ("*Camellia sinensis*" de la famille des *Theaceae*) dont l'oxydation a été arrêtée après la cueillette par diverses méthodes (chauffage, dessiccation, etc...).
"**Vitamine A**" désigne une vitamine qui existe sous différentes formes (rétinal, acide rétinoïque, rétinol).
"**Vitamine E**" désigne une vitamine qui existe sous différentes formes (α-tocophérol, γ-tocophérol, ...).
"**Yerba Maté**" désigne une plante ("*Ilex paraguariensis*" de la famille des *Aquifoliaceae*) dont l'infusion des feuilles dans de l'eau chaude permet d'obtenir une boisson appelée maté.
"**Zéaxanthine**" désigne le composé chimique de formule : 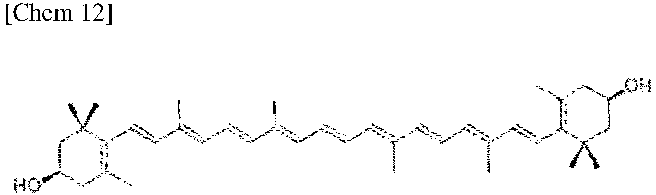
"Zinc" désigne un oligo-élément.

### DESCRIPTION DÉTAILLÉE

### Composition nutraceutique

### Principes actifs principaux

La présente invention concerne une composition nutraceutique, également nommée composition dans la présente description, comprenant du *Bacopa monnieri* et du matcha. De préférence, ladite composition nutraceutique comprend un extrait de *Bacopa monnieri* et du matcha.

La composition nutraceutique selon la présente invention comprend : entre 1,6 et 9,2 mg, préférentiellement entre 1,6 et 8,3 mg, plus préférentiellement entre 1,6 et 7 mg, et encore mieux entre 2 et 7 mg d'extrait de *Bacopa monnieri* par gramme de ladite composition. En d'autres termes, la composition nutraceutique comprend : entre 0,16 % et 0,92 % en poids, préférentiellement entre 0,16 % et 0,83 % en poids, plus préférentiellement entre 0,16 % et 0,7 % en poids, et encore mieux entre 0,2 % et 0,7 % en poids d'extrait de *Bacopa monnieri* par rapport au poids total de ladite composition.

Dans un premier mode de réalisation, la composition nutraceutique selon la présente invention comprend entre 2 et 5 mg d'extrait de *Bacopa monnieri* par gramme de ladite composition. En d'autres termes, la composition nutraceutique comprend 0,2 % et 0,5 % en poids d'extrait de *Bacopa monnieri* par rapport au poids total de ladite composition.

Dans un second mode de réalisation, la composition nutraceutique selon la présente invention comprend entre 5 et 7 mg d'extrait de *Bacopa monnieri* par gramme de ladite composition. En d'autres termes, la composition nutraceutique comprend 0,5 % et 0,7 % en poids d'extrait de *Bacopa monnieri* par rapport au poids total de ladite composition.

Dans un mode de réalisation, la composition nutraceutique selon la présente invention comprend : entre 95 et 550 mg, préférentiellement entre 95 et 500 mg, plus préférentiellement entre 95 et 420 mg, et encore mieux entre 120 et 420 mg d'extrait de *Bacopa monnieri.*

La composition nutraceutique selon la présente invention comprend : entre 1,2 et 9,1 mg, préférentiellement entre 1,2 et 8,3 mg, plus préférentiellement entre 1,2 et 7 mg, et encore mieux entre 5 et 7 mg de matcha par gramme de ladite composition. En d'autres termes, la composition nutraceutique comprend : entre 0,12 % et 0,91 % en poids, préférentiellement entre 0,12 % et 0,83 % en poids, plus préférentiellement entre 0,12 % et 0,7 % en poids, et encore mieux entre 0,5 % et 0,7 % en poids de matcha par rapport au poids total de ladite composition.

Dans un mode de réalisation, la composition nutraceutique selon la présente invention comprend : entre 70 et 550 mg, préférentiellement entre 70 et 500 mg, plus préférentiellement entre 70 et 420 mg, et encore mieux entre 300 et 420 mg de matcha.

De manière préférée, la composition nutraceutique comprend du *Bacopa monnieri* sous la forme de feuilles sèches de *Bacopa monnieri* ou sous la forme d'un extrait de *Bacopa monnieri,* de préférence sous la forme d'un extrait de feuilles de *Bacopa monnieri.*

La composition nutraceutique de l'invention peut éventuellement comprendre en outre un extrait de *Rhodiola rosea* et/ou du Yerba Maté. En d'autres termes, la composition nutraceutique peut éventuellement comprendre en outre un extrait de *Rhodiola rosea* ou du Yerba Maté ou un mélange d'extrait de *Rhodiola rosea* et du Yerba maté. Préférentiellement, la composition nutraceutique comprend en outre un mélange d'extrait de *Rhodiola rosea* et du Yerba Maté.

Dans un mode de réalisation, la composition nutraceutique comprend en tant que principes actifs principaux : du *Bacopa monnieri,* du matcha et un extrait de *Rhodiola rosea.* De préférence, la composition nutraceutique comprend en tant que principes actifs : un extrait de *Bacopa monnieri,* du matcha et un extrait de *Rhodiola rosea.*

Dans un autre mode de réalisation, la composition nutraceutique comprend en tant que principes actifs principaux : du *Bacopa monnieri,* du matcha et du Yerba Maté. De préférence, la composition nutraceutique comprend en tant que principes actifs principaux : un extrait de *Bacopa monnieri,* du matcha et un extrait de Yerba Maté.

Dans un mode de réalisation préféré, la composition nutraceutique comprend en tant que principes actifs principaux : du *Bacopa monnieri,* du matcha, un extrait de *Rhodiola rosea* et du Yerba Maté. De préférence, la composition nutraceutique comprend en tant que principes actifs principaux : un extrait de *Bacopa monnieri,* du matcha, un extrait de *Rhodiola rosea* et un extrait de Yerba Maté.

Dans un mode de réalisation, la composition nutraceutique comprend en outre un extrait de *Rhodiola rosea,* qui peut être sous la forme d'un extrait liquide des racines de *Rhodiola rosea* ou sous la forme d'un extrait sec des racines de *Rhodiola rosea.*

Dans un mode de réalisation, la composition nutraceutique comprend en outre : entre 0,92 et 5,8 mg, préférentiellement entre 2,5 et 4,2 mg, et plus préférentiellement entre 3,0 et 3,6 mg d'extrait de *Rhodiola rosea* par gramme de ladite composition. En d'autres termes, la composition nutraceutique peut comprendre en outre : entre 0,092 % et 0,58 % en poids, préférentiellement entre 0,25 % et 0,42 % en poids, et plus préférentiellement entre 0,3 % et 0,36 % en poids d'extrait de *Rhodiola rosea* par rapport au poids total de ladite composition.

Dans un mode de réalisation, la composition nutraceutique comprend en outre : entre 55 et 350 mg, préférentiellement entre 150 et 250 mg, et plus préférentiellement entre 180 et 220 mg d'extrait de *Rhodiola rosea.*

Dans un mode de réalisation, la composition nutraceutique comprend en outre du Yerba Maté, qui peut être sous la forme d'un extrait sec de feuilles de Yerba Maté, sous la forme de feuilles sèches de Yerba Maté ou sous la forme d'un extrait liquide de Yerba Maté.

Dans un mode de réalisation, la composition nutraceutique comprend en outre : entre 5 et 33,3 mg, préférentiellement entre 10 et 20 mg, et plus préférentiellement entre 13 et 17 mg de Yerba Maté, de préférence sous la forme de feuilles sèches de Yerba Maté, par gramme de ladite composition. En d'autres termes, la composition nutraceutique peut comprendre en outre : entre 0,5 % et 3,33 % en poids, préférentiellement entre 1 % et 2 % en poids, et plus préférentiellement entre 1,3 % et 1,7 % en poids de Yerba Maté, de préférence sous la forme de feuilles sèches de Yerba Maté, par rapport au poids total de ladite composition.

Dans un mode de réalisation, la composition nutraceutique comprend en outre : entre 300 et 2000 mg, préférentiellement entre 600 et 1200 mg, et plus préférentiellement entre 780 et 1020 mg de Yerba Maté, de préférence sous la forme de feuilles sèches de Yerba Maté.

Dans un mode de réalisation préféré, la composition nutraceutique comprend en outre : entre 0,092 % et 0,58 % en poids, préférentiellement entre 0,25 % et 0,42 % en poids, et plus préférentiellement entre 0,3 % et 0,36 % en poids d'extrait de *Rhodiola rosea* par rapport au poids total de ladite composition ; et entre 0,5 % et 3,33 % en poids, préférentiellement entre 1 % et 2 % en poids, et plus préférentiellement entre 1,3 % et 1,7 % en poids de Yerba Maté, de préférence sous la forme de feuilles sèches de Yerba Maté, par rapport au poids total de ladite composition.

### Principes actifs additionnels

Dans un mode de réalisation, la composition nutraceutique de l'invention comprend en outre au moins un principe actif additionnel, différent des principes actifs principaux définis ci-avant, choisi dans le groupe constitué de : caféine, L-théanine, lutéine, zéaxanthine, *Panax ginseng*, *Curcuma longa*, Cordyceps, Ashwaganda, créatine, *Panax quinquefolius,* zinc, *Ocimum sanctum,* safran, *Ginkgo biloba,* extrait *d'Andrographis paniculata,* cannabidiol (CBD) et des vitamines telles que riboflavine (vitamine B2), biotine (vitamine B8), acide folique (vitamine B9), vitamine A et vitamine E.

De préférence, la composition nutraceutique de l'invention peut éventuellement comprendre au moins un principe actif additionnel comprenant au moins un composé choisi dans le groupe constitué de : caféine, L-théanine, lutéine, zéaxanthine, *Panax ginseng*, *Curcuma longa*, Cordyceps, Ashwaganda, créatine, *Panax quinquefolius,* zinc, *Ocimum sanctum,* safran, *Ginkgo biloba,* extrait d'*Andrographis paniculata,* cannabidiol (CBD) et au moins une vitamine choisie dans le groupe constitué de riboflavine (vitamine B2), biotine (vitamine B8), acide folique (vitamine B9), vitamine A et vitamine E.

Préférentiellement, la composition nutraceutique comprend en outre de la caféine et de la L-théanine. Plus préférentiellement, la composition nutraceutique comprend en outre de la caféine, de la L-théanine, de la riboflavine (vitamine B2), de la biotine (vitamine B8) et de l'acide folique (vitamine B9).

Dans un mode de réalisation, la composition nutraceutique comprend en outre de la caféine. Le Guarana peut être utilisé comme source de caféine, par exemple sous la forme d'un extrait de graines de Guarana.

Dans un mode de réalisation, la composition nutraceutique comprend en outre : entre 55 et 683 µg, préférentiellement entre 92 et 683 µg, et plus préférentiellement entre 133 et 633 µg de caféine provenant du Guarana par gramme de ladite composition. En d'autres termes, la composition nutraceutique peut comprendre en outre : entre 0,0055 % et 0,0683 % en poids, préférentiellement entre 0,0092 % et 0,0683 % en poids, et plus préférentiellement entre 0,0133 % et 0,0633 % en poids de caféine provenant du Guarana par rapport au poids total de ladite composition.

Dans un mode de réalisation, la composition nutraceutique comprend en outre : entre 3,3 et 41 mg, préférentiellement entre 5,5 et 41 mg, et plus préférentiellement entre 8 et 38 mg de caféine provenant du Guarana.

Dans un mode de réalisation, la composition nutraceutique comprend en outre : entre 0,8 et 6,7 mg, préférentiellement entre 3 et 6,6 mg, et plus préférentiellement entre 3 et 6,3 mg d'extrait de Guarana contenant 7 % de caféine par gramme de ladite composition. En d'autres termes, la composition nutraceutique peut comprendre en outre : entre 0,08 % nutraceutique et 0,67 % en poids, préférentiellement entre 0,3 % et 0,66 % en poids, et plus préférentiellement entre 0,3 % et 0,63 % en poids d'extrait de Guarana contenant 7 % de caféine par rapport au poids total de ladite composition.

Dans un mode de réalisation, la composition nutraceutique comprend en outre : entre 48 et 400 mg, préférentiellement entre 180 et 395 mg, et plus préférentiellement entre 180 et 380 mg d'extrait de Guarana contenant 7 % de caféine.

Dans un mode de réalisation, la composition nutraceutique comprend en outre : entre 0,8 et 6,7 mg, préférentiellement entre 1 et 6,6 mg, et plus préférentiellement entre 1,5 et 6,3 mg d'extrait de Guarana contenant 10 % de caféine par gramme de ladite composition. En d'autres termes, la composition nutraceutique peut comprendre en outre : entre 0,08 % et 0,67 % en poids, préférentiellement entre 0,1 % et 0,66 % en poids, et plus préférentiellement entre 0,15 % et 0,63 % en poids d'extrait de Guarana contenant 10 % de caféine par rapport au poids total de ladite composition.

Dans un mode de réalisation, la composition nutraceutique comprend en outre : entre 48 et 400 mg, préférentiellement entre 60 et 395 mg, et plus préférentiellement entre 90 et 380 mg d'extrait de Guarana contenant 10 % de caféine.

Dans un mode de réalisation, la composition nutraceutique comprend en outre de la L-théanine. La L-théanine peut être extraite de feuilles de *Camellia sinensis* ou peut être d'origine synthétique.

Dans un mode de réalisation, la composition nutraceutique comprend en outre : entre 0,33 et 2,5 mg, préférentiellement entre 0,58 et 0,92 mg, et plus préférentiellement entre 0,65 et 0,85 mg de L-théanine par gramme de ladite composition. En d'autres termes, la composition nutraceutique peut comprendre en outre : entre 0,033 % et 0,25 % en poids, préférentiellement entre 0,058 % et 0,092 % en poids, et plus préférentiellement entre 0,065 % et 0,085 % en poids de L-théanine par rapport au poids total de ladite composition.

Dans un mode de réalisation, la composition nutraceutique comprend en outre : entre 20 et 155 mg, préférentiellement entre 35 et 55 mg, et plus préférentiellement entre 40 et 51 mg de L-théanine.

Dans un mode de réalisation préféré, la composition nutraceutique comprend en outre : entre 0,08 % nutraceutique et 0,67 % en poids, préférentiellement entre 0,3 % et 0,66 % en poids, et plus préférentiellement entre 0,53 % et 0,63 % en poids d'extrait de Guarana contenant 7 % de caféine par rapport au poids total de ladite composition ; et entre 0,033 % et 0,25 % en poids, préférentiellement entre 0,058 % et 0,092 % en poids, et plus préférentiellement entre 0,065 % et 0,085 % en poids de L-théanine par rapport au poids total de ladite composition.

Dans un mode de réalisation, la composition nutraceutique comprend en outre de la lutéine.

Dans un mode de réalisation, la composition nutraceutique comprend en outre de la zéaxanthine.

Dans un mode de réalisation, la composition nutraceutique comprend en outre du *Panax ginseng.*

Dans un mode de réalisation, la composition nutraceutique comprend en outre du *Curcuma longa.*

Dans un mode de réalisation, la composition nutraceutique comprend en outre du Cordyceps.

Dans un mode de réalisation, la composition nutraceutique comprend en outre de l'Ashwaganda.

Dans un mode de réalisation, la composition nutraceutique comprend en outre de la créatine.

Dans un mode de réalisation, la composition nutraceutique comprend en outre du *Panax quinquefolius.*

Dans un mode de réalisation, la composition nutraceutique comprend en outre du zinc.

Dans un mode de réalisation, la composition nutraceutique comprend en outre de l'*Ocimum sanctum.*

Dans un mode de réalisation, la composition nutraceutique comprend en outre du safran.

Dans un mode de réalisation, la composition nutraceutique comprend en outre du *Ginkgo biloba.*

Dans un mode de réalisation, la composition nutraceutique comprend en outre de l'extrait d'*Andrographis paniculata.*

Dans un mode de réalisation, la composition nutraceutique comprend en outre du cannabidiol (CBD).

Dans un mode de réalisation, la composition nutraceutique comprend en outre au moins un principe actif additionnel qui est une vitamine. En d'autres termes, dans ce mode de réalisation, la composition nutraceutique comprend en outre au moins une vitamine, telle que la riboflavine (vitamine B2), la biotine (vitamine B8), l'acide folique (vitamine B9), la vitamine A ou la vitamine E.

Dans un mode de réalisation, la composition nutraceutique comprend en outre de la riboflavine (vitamine B2), de la biotine (vitamine B8) et/ou de l'acide folique (vitamine B9). En d'autres termes, dans ce mode de réalisation, la composition nutraceutique comprend en outre de la riboflavine (vitamine B2), de la biotine (vitamine B8) ou de l'acide folique (vitamine B9) ou un mélange de riboflavine (vitamine B2), de biotine (vitamine B8) et d'acide folique (vitamine B9). Préférentiellement, la composition nutraceutique comprend en outre un mélange de riboflavine (vitamine B2), de biotine (vitamine B8) et d'acide folique (vitamine B9).

Dans un mode de réalisation, la composition nutraceutique comprend en outre de la riboflavine (vitamine B2).

Dans un mode de réalisation, la composition nutraceutique comprend en outre : entre 8,3 et 33 µg, préférentiellement entre 13 et 29 µg, et plus préférentiellement entre 17 et 25 µg de riboflavine (vitamine B2) par gramme de ladite composition.

Dans un mode de réalisation, la composition nutraceutique comprend en outre : entre 500 et 2000 µg, préférentiellement entre 780 et 1740 µg, et plus préférentiellement entre 1020 et 1500 µg de riboflavine (vitamine B2).

Dans un mode de réalisation, la composition nutraceutique comprend en outre de la biotine (vitamine B8).

Dans un mode de réalisation, la composition nutraceutique comprend en outre : entre 0,67 et 2,5 µg, préférentiellement entre 0,67 et 1,6 µg, plus préférentiellement entre 0,67 et 1,4 µg et encore mieux entre 0,8 et 1,4 µg de biotine (vitamine B8) par gramme de ladite composition.

Dans un mode de réalisation, la composition nutraceutique comprend en outre : entre 40 et 150 µg, préférentiellement entre 40 et 100 µg, plus préférentiellement entre 40 et 85 µg et encore mieux entre 50 et 85 µg de biotine (vitamine B8).

Dans un mode de réalisation, la composition nutraceutique comprend en outre de l'acide folique (vitamine B9).

Dans un mode de réalisation, la composition nutraceutique comprend en outre : entre 1,5 et 10 µg, préférentiellement entre 2,1 et 3,1 µg, et plus préférentiellement entre 2,4 et 3,1 µg d'acide folique (vitamine B9) par gramme de ladite composition.

Dans un mode de réalisation, la composition nutraceutique comprend en outre : entre 90 et 600 µg, préférentiellement entre 126 et 186 µg, et plus préférentiellement entre 144 et 186 µg d'acide folique (vitamine B9).

Dans un mode de réalisation, la composition nutraceutique comprend en outre de la vitamine A.

Dans un mode de réalisation, la composition nutraceutique comprend en outre de la vitamine E.

Dans un mode de réalisation encore plus préféré, la composition nutraceutique comprend en outre : entre 0,08 % nutraceutique et 0,67 % en poids, préférentiellement entre 0,3 % et 0,66 % en poids, et plus préférentiellement entre 0,53 % et 0,63 % en poids d'extrait de Guarana contenant 7 % de caféine par rapport au poids total de ladite composition ; entre 0,033 % et 0,25 % en poids, préférentiellement entre 0,058 % et 0,092 % en poids, et plus préférentiellement entre 0,065 % et 0,085 % en poids de L-théanine par rapport au poids total de ladite composition ; de la riboflavine (vitamine B2), de la biotine (vitamine B8) et de l'acide folique (vitamine B9).

### Excipients

Dans un mode de réalisation, la composition nutraceutique comprend en outre au moins un excipient choisi dans le groupe constitué d'extrait naturel aromatique de pêche, d'extrait naturel aromatique d'abricot, d'huile essentielle de menthe des champs, de miel, de stévia, de sirop de dattes, de chlorophylle cuivrique, de pectine, d'agar agar, d'hydroxypropylméthylcellulose, de cellulose, d'acide stéarique, de cellulose microcristalline, de maltitol, de sorbitol, de mannitol, de polyéthylène glycol, de sucralose, de polysorbate 80, de diméthylpolysiloxane, de sorbate de potassium, de benzoate de sodium et d'acide citrique.

Dans un mode de réalisation, la composition nutraceutique comprend en outre de l'extrait naturel aromatique de pêche.

Dans un mode de réalisation, la composition nutraceutique comprend en outre de l'extrait naturel aromatique d'abricot.

Dans un mode de réalisation, la composition nutraceutique comprend en outre de l'huile essentielle de menthe des champs.

Dans un mode de réalisation, la composition nutraceutique comprend en outre du miel.

Dans un mode de réalisation, la composition nutraceutique comprend en outre de la stévia.

Dans un mode de réalisation, la composition nutraceutique comprend en outre de la chlorophylle cuivrique.

Dans un mode de réalisation, la composition nutraceutique comprend en outre du sirop de dattes.

Dans un mode de réalisation, la composition nutraceutique comprend en outre de la pectine.

Dans un mode de réalisation, la composition nutraceutique comprend en outre de l'agar agar.

Dans un mode de réalisation, la composition nutraceutique comprend en outre de l'hydroxypropylméthylcellulose.

Dans un mode de réalisation, la composition nutraceutique comprend en outre de la cellulose.

Dans un mode de réalisation, la composition nutraceutique comprend en outre de l'acide stéarique.

Dans un mode de réalisation, la composition nutraceutique comprend en outre de la cellulose microcristalline.

Dans un mode de réalisation, la composition nutraceutique comprend en outre du maltitol.

Dans un mode de réalisation, la composition nutraceutique comprend en outre du sorbitol.

Dans un mode de réalisation, la composition nutraceutique comprend en outre du mannitol.

Dans un mode de réalisation, la composition nutraceutique comprend en outre du polyéthylène glycol.

Dans un mode de réalisation, la composition nutraceutique comprend en outre de la sucralose.

Dans un mode de réalisation, la composition nutraceutique comprend en outre du polysorbate 80.

Dans un mode de réalisation, la composition nutraceutique comprend en outre du diméthylpoly siloxane.

Dans un mode de réalisation, la composition nutraceutique comprend en particulier au moins un conservateur choisi dans le groupe constitué de sorbate de potassium, benzoate de sodium, et acide citrique.

Dans un mode de réalisation, la composition nutraceutique comprend en outre du sorbate de potassium.

Dans un mode de réalisation, la composition nutraceutique comprend en outre du benzoate de sodium.

Dans un mode de réalisation, la composition nutraceutique comprend en outre du sorbate de potassium et du benzoate de sodium.

Dans un mode de réalisation, la composition nutraceutique comprend en outre de l'acide citrique.

Dans un mode de réalisation, la composition nutraceutique est une solution et la quantité d'acide citrique est choisie de manière à obtenir un pH de ladite solution inférieur à 5.

### Formulation

Dans un mode de réalisation, la composition nutraceutique selon l'invention se présente sous une des formes suivantes : gélules, comprimés, capsules molles, solution concentrée à diluer, poudres à disperser, solution, solution buvable, solution aqueuse, solution aqueuse buvable, poudres à solubiliser, gel, bonbons, barre de céréales.

Dans un mode de réalisation, la composition nutraceutique selon l'invention se présente sous la forme de gélules.

Dans un mode de réalisation, la composition nutraceutique selon l'invention se présente sous la forme de comprimés.

Dans un mode de réalisation, la composition nutraceutique selon l'invention se présente sous la forme de capsules molles.

Dans un mode de réalisation, la composition nutraceutique selon l'invention se présente sous la forme d'une solution concentrée à diluer.

Dans un mode de réalisation, la composition nutraceutique selon l'invention se présente sous la forme de poudre à disperser.

Dans un mode de réalisation, la composition nutraceutique selon l'invention se présente sous la forme d'une solution.

Dans un mode de réalisation, la composition nutraceutique selon l'invention se présente sous la forme d'une solution buvable.

Préférentiellement, la composition nutraceutique selon l'invention se présente sous la forme d'une solution aqueuse, d'une solution aqueuse buvable, de poudre à solubiliser, de gel, de bonbons ou de barre de céréales.

### Effets obtenus

Les inventeurs ont découvert des effets surprenants lorsque le matcha est associé à un extrait de *Bacopa monnieri* :
- un état émotionnel plus calme,
- une augmentation de la mémoire de travail,
- une diminution de l'état de fatigue général, de la fatigue physique et mentale et de l'état de fatigue impactant le nombre quotidien d'activités,
- une augmentation de la motivation, et
- une augmentation de la concentration.

### Composition nutraceutique pour son utilisation

La présente invention a également pour objet la composition nutraceutique selon la présente invention pour son utilisation en tant que complément alimentaire, de préférence pour augmenter l'énergie et les fonctions cognitives du sujet auquel est administré la composition (en particulier la concentration et la mémoire) tout en procurant un effet calmant qui consiste en une diminution du stress et de l'anxiété.

Dans un mode de réalisation, la composition selon l'invention est utilisée en tant que complément alimentaire, de préférence pour augmenter l'énergie et les fonctions cognitives du sujet auquel est administré la composition (en particulier la concentration et la mémoire) tout en procurant un effet calmant qui consiste en une diminution du stress et de l'anxiété.

Dans un mode de réalisation, la composition nutraceutique est administrée par voie orale.

Dans un mode de réalisation, la composition nutraceutique est administrée quand le sujet veut obtenir une augmentation de sa productivité.

De manière préférée, la composition nutraceutique est administrée une fois par jour.

De manière préférée, la composition nutraceutique est administrée pendant trois mois.

### Procédé de préparation de la composition nutraceutique

La présente invention a également pour objet un procédé de préparation d'une composition nutraceutique selon la présente invention, comprenant les étapes suivantes :
a) mélanger ensemble entre 0,16 % et 0,92 % en poids d'un extrait de *Bacopa monnieri* par rapport au poids total de ladite composition et entre 0,12 % et 0,91 % en poids de matcha par rapport au poids total de ladite composition,
b) éventuellement, ajouter un extrait de *Rhodiola rosea* et/ou du Yerba Maté,
c) éventuellement ajouter au moins un principe actif additionnel tel que décrit ci-avant et/ou au moins un excipient tel que décrit ci-avant, et
d) éventuellement mélanger l'ensemble obtenu à l'une des éventuelles étapes b) ou c).

Dans un mode de réalisation, le procédé de préparation d'une composition nutraceutique selon la présente invention, ladite composition nutraceutique étant sous forme d'une solution buvable, comprend les étapes suivantes :
a) préparer un milieu véhicule liquide,
b) mélanger ensemble entre 0,16 % et 0,92 % en poids d'un extrait de *Bacopa monnieri* par rapport au poids total de ladite composition et entre 0,12 % et 0,91 % en poids de matcha par rapport au poids total de ladite composition,
c) ajouter de manière progressive le milieu véhicule liquide obtenu à l'étape a) sur le mélange obtenu à l'étape b),
d) éventuellement, ajouter un extrait de *Rhodiola rosea* et/ou du Yerba Maté,
e) éventuellement, ajouter au moins un principe actif additionnel tel que décrit ci-avant et/ou au moins un excipient tel que décrit ci-avant, et
f) mélanger l'ensemble obtenu à l'étape c) ou à l'une des éventuelles étapes d) et e) pour obtenir la composition nutraceutique.

Dans un autre mode de réalisation, le procédé de préparation d'une composition nutraceutique selon la présente invention, ladite composition nutraceutique étant sous forme d'une solution buvable, comprend les étapes suivantes :
a) préparer un milieu véhicule liquide,
b) mélanger ensemble entre 0,16 % et 0,92 % en poids d'un extrait de *Bacopa monnieri* par rapport au poids total de ladite composition et entre 0,12 % et 0,91 % en poids de matcha par rapport au poids total de ladite composition,
c) éventuellement, ajouter un extrait de *Rhodiola rosea* et/ou du Yerba Maté au mélange obtenu à l'étape b),
d) éventuellement, ajouter au moins un principe actif additionnel tel que décrit ci-avant au mélange obtenu à l'étape b) ou à l'éventuelle étape c),
e) ajouter de manière progressive le milieu véhicule liquide obtenu à l'étape a) sur le mélange obtenu à l'étape b) ou sur le mélange obtenu à l'une des éventuelles étapes c) et d),
f) éventuellement, ajouter au moins un excipient tel que décrit ci-avant à l'ensemble obtenu à l'étape e), et
g) mélanger l'ensemble obtenu à l'étape e) ou l'ensemble obtenu à l'éventuelle étape f) pour obtenir la composition nutraceutique.

### BRÈVE DESCRIPTION DES FIGURES

**[****Fig. 1] Figure 1** est un graphique montrant le score moyen au test STAI-Y Trait (State Trait Anxiety Inventory) avant et après la consommation de la composition décrite dans l'exemple 1 sous forme de solution aqueuse de 60 mL une fois par jour pendant 10 jours.
**[****Fig. 2] Figure 2** est un graphique montrant le nombre d'erreurs moyen au test DOT-A (Adaptative Digit Ordering Test) avant et après la consommation de la composition décrite dans l'exemple 1 sous forme de solution aqueuse de 60 mL une fois par jour pendant 10 jours.
**[****Fig. 3] Figure 3** est un graphique montrant les scores moyens au test MFI (Multidimensional Fatigue Inventory) avant et après la consommation de la composition décrite dans l'exemple 1 sous forme de solution aqueuse de 60 mL une fois par jour pendant 10 jours.
**[****Fig. 4] Figure 4** est un graphique montrant les scores moyens au test ANT (Attention Network Test) avant et après la consommation de la composition décrite dans l'exemple 1 sous forme de solution aqueuse de 60 mL une fois par jour pendant 10 jours.

### EXEMPLES

La présente invention se comprendra mieux à la lecture des exemples suivants qui illustrent non-limitativement l'invention.

Des exemples de composition nutraceutique selon l'invention (sans excipients) ont été préparés selon les tableaux des exemples 1 à 11 ci-après. Un procédé de préparation de la composition nutraceutique décrite dans l'exemple 2 (avec excipients) est présenté dans l'exemple 12.

### Exemple 1 : exemple de composition nutraceutique selon l'invention

**[Table 1]**

| **Ingrédient** | **Description** | **Source de l'ingrédient** | **Quantité de l'ingrédient (par gramme de composition)** | **Quantité de l'ingrédient (% en poids par rapport au poids total de composition)** |
|---|---|---|---|---|
| Extrait de *Bacopa monnieri* | Comprend 20 % en poids de bacoside | Poudre extrait des feuilles | 6,7 mg/g | 0,67 % |
| Matcha | | Poudre de thé vert moulu | 6,7 mg/g | 0,67 % |
| Extrait de *Rhodiola rosea* | Comprend 3 % en poids de rosavin, et 1 % en poids de salidroside | Poudre extrait des racines | 3,3 mg/g | 0,33 % |
| Yerba Maté | Comprend 2 % en poids de caféine | Feuilles | 15 mg/g | 1,5 % |
| Guarana | Comprend 7 % en poids de caféine | Poudre extrait de graines de Guarana | 5,8 mg/g | 0,58 % |
| L-théanine | | Extrait de *Camellia sinensis* | 0,75 mg/g | 0,075 % |
| Eau | | | Qsp 1g | Qsp 100 % |

### Exemple 2 : exemple de composition nutraceutique selon l'invention

**[Table 2]**

| **Ingrédient** | **Description** | **Source de l'ingrédient** | **Quantité de l'ingrédient (par gramme de composition)** | **Quantité de l'ingrédient (% en poids par rapport au poids total de composition)** |
|---|---|---|---|---|
| Extrait de *Bacopa monnieri* | Comprend 20 % en poids de bacoside | Poudre extrait des feuilles | 6,7 mg/g | 0,67 % |
| Matcha | | Poudre de thé vert moulu | 6,7 mg/g | 0,67 % |
| Extrait de *Rhodiola rosea* | Comprend 3 % en poids de rosavin, et 1 % en poids de salidroside | Poudre extrait des racines | 3,3 mg/g | 0,33 % |
| Yerba Maté | Comprend 2 % en poids de caféine | Feuilles | 15 mg/g | 1,5 % |
| Riboflavine (vitamine B2) | | Fécule de maïs | 21 µg/g | 0,0021 % |
| Biotine (vitamine B8) | | Synthétique | 1,25 µg/g | 0,000125 % |
| Acide folique (vitamine B9) | | Synthétique | 2,6 µg/g | 0,00026 % |
| Guarana | Comprend 7 % en poids de caféine | Poudre extrait de graines de Guarana | 5,8 mg/g | 0,58 % |
| L-théanine | | Extrait de *Camellia sinensis* | 0,75 mg/g | 0,075 % |
| Milieu véhicule liquide | | | Qsp 1g | Qsp 100 % |

### Exemple 3 : exemple de composition nutraceutique selon l'invention

**[Table 3]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,5 % |
| Lutéine | | | 0,016 % |
| Zéaxanthine | | | 0,01% |
| Vitamine A | | | 0,001 % |
| Milieu véhicule liquide ou sec | | | Qsp 100% |

### Exemple 4 : exemple de composition nutraceutique selon l'invention

**[Table 4]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,6 % |
| *Panax ginseng* | | | 0,4 % |
| Milieu véhicule liquide ou sec | | | Qsp 100% |

### Exemple 5 : exemple de composition nutraceutique selon l'invention

**[Table 5]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,6 % |
| *Curcuma longa* | | | 0,1 % |
| Cordyceps | | | 0,5 % |
| Ashwaganda | | | 0,7 % |
| Milieu véhicule liquide ou sec | | | Qsp 100% |

### Exemple 6 : exemple de composition nutraceutique selon l'invention

**[Table 6]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,5 % |
| Créatine | | | 0,18 % |
| *Panax quinquefolius* | | | 0,6 % |
| Milieu véhicule liquide ou sec | | | Qsp 100% |

### Exemple 7 : exemple de composition nutraceutique selon l'invention

**[Table 7]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,67 % |
| Riboflavine (vitamine B2) | | Fécule de maïs | 0,002 % |
| Biotine (vitamine B8) | | Synthétique | 0,0001 % |
| Acide folique (vitamine B9) | | Synthétique | 0,0003 % |
| Vitamine A | | | 0,001 % |
| Vitamine E | | | 0,025 % |
| L-théanine | | Feuilles de *Camellia sinensis* | 0,085 % |
| Lutéine | | | 0,016 % |
| Zinc | | | 0,016 % |
| Milieu véhicule liquide ou sec | | | Qsp 100% |

### Exemple 8 : exemple de composition nutraceutique selon l'invention

**[Table 8]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,6 % |
| *Ocimum sanctum* | | | 0,33 % |
| Milieu véhicule liquide ou sec | | | Qsp 100% |

### Exemple 9 : exemple de composition nutraceutique selon l'invention

**[Table 9]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,2 % |
| Matcha | | Poudre de thé vert moulu | 0,67 % |
| Extrait de *Rhodiola rosea* | Comprend 3 % en poids de rosavin, et 1 % en poids de salidroside | Poudre extrait des racines | 0,33 % |
| Safran | | Extrait de Safran (stigmates) | 0,05 % |
| Milieu véhicule liquide ou sec | | | Qsp 100% |

### Exemple 10 : exemple de composition nutraceutique selon l'invention

**[Table 10]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside. | Poudre extrait des feuilles | 0,2 % |
| Matcha | | Poudre de thé vert moulu | 0,5 % |
| Cannabidiol (CBD) | | | 0,03 % |
| Milieu véhicule liquide ou sec | | | Qsp 100% |

### Exemple 11 : exemple de composition nutraceutique selon l'invention

**[Table 11]**

| **Ingrédient** | **Description** | **Source de l'ingrédient** | **Quantité de l'ingrédient (% en poids par rapport au poids total de composition)** |
|---|---|---|---|
| Extrait de *Bacopa monnieri* | Comprend 20 % en poids de bacoside | Poudre extrait des feuilles | 0,3 % |
| Matcha | | Poudre de thé vert moulu | 0,6 % |
| *Ginkgo biloba* | | | 0,3 % |
| Milieu véhicule liquide ou sec | | | Qsp 100% |

### Exemple 12 : Procédé de préparation d'un complément alimentaire.

La composition décrite dans l'exemple 2 a été préparée avec des excipients sous la forme d'une solution aqueuse buvable selon un procédé dont les étapes sont détaillées ci-dessous.

### Préparation du milieu véhicule liquide

Une infusion de Yerba Maté, aussi appelée extrait de Yerba Maté, est préparée en infusant pendant 15 minutes 15 grammes de feuilles de Yerba Maté dans 1 L d'eau puis en filtrant les solides.

Un arôme de menthe est préparé en mélangeant 5 mL de l'infusion de Yerba Maté et une goutte d'huile essentielle de menthe des champs.

Le milieu véhicule liquide est obtenu en mélangeant à l'infusion de Yerba Maté une goutte d'extrait naturel aromatique de pêche, une goutte d'extrait naturel aromatique d'abricot et 0,5 mL de l'arôme de menthe.

### Mixage des principes actifs

Les principes actifs décrits dans la [Table 2] (à l'exception des vitamines et du Yerba Maté) sont mélangés ensemble dans un agitateur.

Les vitamines sont ajoutées au mélange précédent et le tout est mélangé dans un agitateur.

### Ajout du milieu véhicule liquide sur le mélange de principes actifs mixé

Le milieu véhicule liquide est ajouté lentement et de manière progressive au mélange de principes actifs. Le tout est mélangé jusqu'à dissolution maximale des solides et obtention d'une solution homogène.

### Ajout des excipients

Du miel et de la chlorophylle cuivrique sont alors ajoutés à la solution homogène obtenue.

De l'acide citrique est ajouté de manière à obtenir un pH de la solution égal à 4,5.

Du sorbate de potassium et du benzoate de sodium sont ajoutés. Leur quantité doit être comprise entre 0,5 % et 1 % de la composition finale.

### Mélange

Le complément alimentaire est obtenu après mélange et ajustement du pH de la solution à 4 par ajout d'acide citrique.

### Exemple 13 : Test d'évaluation du niveau d'anxiété général.

### Matériel et Méthodes

L'étude a été réalisée sur un échantillon composé de 7 personnes. Chaque participant a consommé la composition décrite dans l'exemple 1 sous forme de solution aqueuse de 60 mL, une fois par jour pendant 10 jours.

Le niveau d'anxiété général des participants a été évalué avec le test STAI-Y Trait (State Trait Anxiety Inventory) au début et à la fin de l'étude.

### Résultats

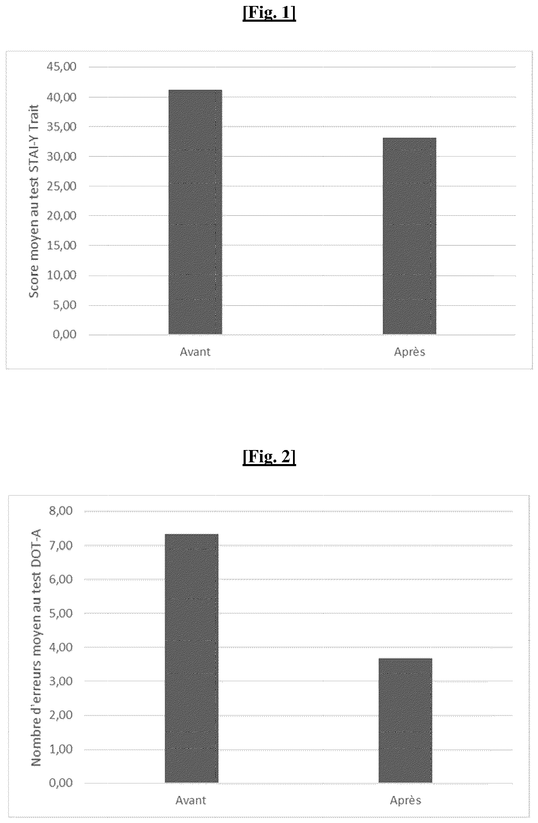

Le score moyen au test STAI-Y Trait à la fin de l'étude a diminué de manière significative, comme montré sur le graphique de la Figure 1. Cela traduit un état émotionnel plus calme des participants résultant de la consommation de la composition décrite dans l'exemple 1.

### Exemple 14 : Test d'évaluation de la mémoire de travail.

### Matériel et Méthodes

L'étude a été réalisée sur un échantillon composé de 6 personnes. Chaque participant a consommé la composition décrite dans l'exemple 1 sous forme de solution aqueuse de 60 mL, une fois par jour pendant 10 jours.

La mémoire de travail a été évaluée avec le test DOT-A (Adaptative Digit Ordering Test) au début et à la fin de l'étude.

### Résultats

Le nombre d'erreurs moyen au test DOT-A à la fin de l'étude a diminué de manière significative, comme montré sur le graphique de la Figure 2. Cela traduit une augmentation de la mémoire de travail des participants résultant de la consommation de la composition décrite dans l'exemple 1.

### Exemple 15 : Test d'évaluation de la fatigue

### Matériel et Méthodes

L'étude a été réalisée sur un échantillon composé de 6 personnes. Chaque participant a consommé la composition décrite dans l'exemple 1 sous forme de solution aqueuse de 60 mL, une fois par jour pendant 10 jours.

La fatigue des sujets a été évaluée avec le test MFI (Multidimensional Fatigue Inventory) au début et à la fin de l'étude.

### Résultats

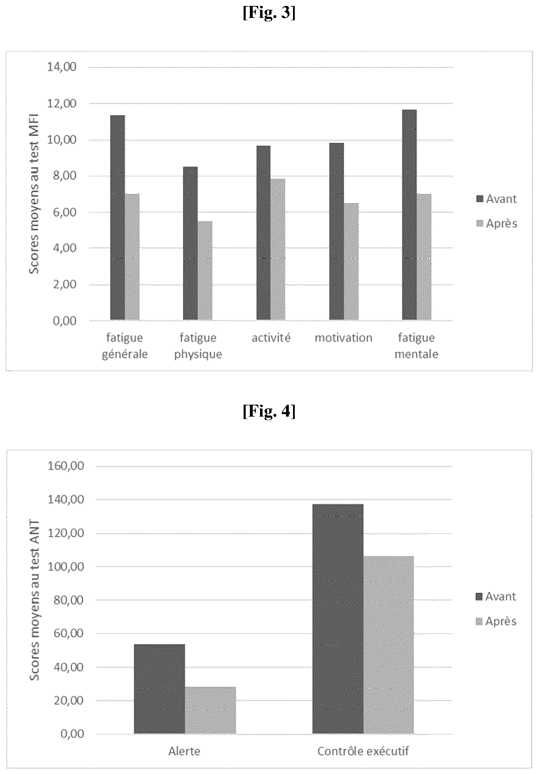

Les scores moyens au test MFI à la fin de l'étude ont diminué de manière significative, comme montré sur le graphique de la Figure 3. Cela traduit chez les participants une diminution de l'état de fatigue général, de la fatigue physique et mentale et de l'état de fatigue impactant le nombre quotidien d'activités. Cela traduit aussi une augmentation de la motivation.

### Exemple 16 : test d'évaluation de la concentration.

### Matériel et Méthodes

L'étude a été réalisée sur un échantillon composé de 6 personnes. Chaque participant a consommé la composition décrite dans l'exemple 1 sous forme de solution aqueuse de 60 mL, une fois par jour pendant 10 jours.

La fatigue des sujets a été évaluée avec le test ANT (Attention Network Test) au début et à la fin de l'étude.

### Résultats

Les scores moyens au test ANT concernant l'état général d'éveil (alerte) et le contrôle exécutif ont diminué de manière significative à la fin de l'étude, comme montré sur le graphique de la Figure 4. Cela traduit une augmentation de la concentration chez les participants, résultant de la consommation de la composition décrite dans l'exemple.

## Revendications

1. Composition nutraceutique comprenant :
- entre 0,16 % et 0,92 % en poids d'un extrait de *Bacopa monnieri,* par rapport au poids total de ladite composition, et
- entre 0,12 % et 0,91 % en poids de matcha, par rapport au poids total de ladite composition.

2. Composition nutraceutique selon la revendication **1**, comprenant en outre un extrait de *Rhodiola rosea* et/ou du Yerba Maté.

3. Composition nutraceutique selon la revendication **1** ou la revendication **2**, comprenant en outre au moins un principe actif additionnel choisi dans le groupe constitué de : caféine, L-théanine, lutéine, zéaxanthine, *Panax ginseng*, *Curcuma longa*, Cordyceps, Ashwaganda, créatine, *Panax quinquefolius,* zinc, *Ocimum sanctum*, safran, *Ginkgo biloba,* extrait d'*Andrographis paniculata,* cannabidiol (CBD), riboflavine (vitamine B2), biotine (vitamine B8), acide folique (vitamine B9), vitamine A et vitamine E.

4. Composition nutraceutique selon l'une quelconque des revendications **1** à **3,** comprenant entre 0,16 % et 0,83 % en poids, préférentiellement entre 0,16 % et 0,7 % en poids, et plus préférentiellement entre 0,2 % et 0,7 % en poids d'extrait de *Bacopa monnieri* par rapport au poids total de ladite composition.

5. Composition nutraceutique selon l'une quelconque des revendications **1** à **4,** comprenant entre 0,12 % et 0,83 % en poids, préférentiellement entre 0,12 % et 0,7 % en poids, et plus préférentiellement entre 0,5 % et 0,7 % en poids de matcha par rapport au poids total de ladite composition.

6. Composition nutraceutique selon l'une quelconque des revendications **2** à **5,** comprenant entre 0,092 % et 0,58 % en poids, préférentiellement entre 0,25 % et 0,42 % en poids, et plus préférentiellement entre 0,3 % et 0,36 % en poids d'extrait de *Rhodiola rosea* par rapport au poids total de ladite composition.

7. Composition nutraceutique selon l'une quelconque des revendications **2** à **6,** comprenant entre 0,5 % et 3,33 % en poids, préférentiellement entre 1 % et 2 % en poids, et plus préférentiellement entre 1,3 % et 1,7 % en poids de Yerba Maté, de préférence sous la forme de feuilles sèches de Yerba Maté, par rapport au poids total de ladite composition.

8. Composition nutraceutique selon l'une quelconque des revendications **1** à **7,** comprenant en outre au moins un excipient choisi dans le groupe constitué d'extrait naturel aromatique de pêche, d'extrait naturel aromatique d'abricot, d'huile essentielle de menthe des champs, de miel, de stévia, de sirop de dattes, de chlorophylle cuivrique, de pectine, d'agar agar, d'hydroxypropylméthylcellulose, de cellulose, d'acide stéarique, de cellulose microcristalline, de maltitol, de sorbitol, de mannitol, de polyéthylène glycol, de sucralose, de polysorbate 80, de diméthylpolysiloxane, de sorbate de potassium, de benzoate de sodium et d'acide citrique.

9. Composition nutraceutique selon l'une quelconque des revendications **1** à **8,** pour son utilisation en tant que complément alimentaire.

10. Composition nutraceutique pour son utilisation selon la revendication **9,** pour augmenter l'énergie et les fonctions cognitives d'un sujet tout en diminuant le stress et l'anxiété.

11. Procédé de préparation d'une composition nutraceutique selon l'une quelconque des revendications **1** à **8**, **caractérisé en ce que** la composition nutraceutique est une solution buvable, et **caractérisé en ce qu'**il comprend les étapes suivantes :
a) préparer un milieu véhicule liquide,
b) mélanger ensemble entre 0,16 % et 0,92 % en poids d'un extrait de *Bacopa monnieri* par rapport au poids total de ladite composition et entre 0,12 % et 0,91 % en poids de matcha par rapport au poids total de ladite composition,
c) ajouter de manière progressive le milieu véhicule liquide obtenu à l'étape a) sur le mélange obtenu à l'étape b),
d) éventuellement, ajouter un extrait de *Rhodiola rosea* et/ou du Yerba Maté,
e) éventuellement, ajouter au moins un principe actif additionnel tel que décrit à la revendication **3** et/ou au moins un excipient tel que décrit à la revendication **8,** et
f) mélanger l'ensemble obtenu à l'étape c) ou à l'une des éventuelles étapes d) et e) pour obtenir la composition nutraceutique.
